# FASCICULE DE BREVET EUROPEEN

(11) **EP 0 709 371 B1**
(45) Date de publication et mention de la délivrance du brevet: **06.05.1998**
(21) Numéro de dépôt: 95402375.0
(22) Date de dépôt: 25.10.1995
(51) Int. Cl.: C07C 259/06, C07C 259/08, C07D 209/12, C07D 333/56, C07D 307/80, C07C 275/64, C07C 335/40, C07C 327/56, A61K 31/165, A61K 31/40, A61K 31/34, A61K 31/38, A61K 31/17

(54) **O-arylméthyl N-(thio)acyl hydroxylamines comme ligands des récepteurs de la mélatonine, leurs procédés de préparation et les compositions pharmaceutiques qui les contiennent**
O-Arylmethyl-N-(thio)acyl-hydroxylamine als Liganden des Melatoninrezeptors, Verfahren zu ihrer Herstellung und sie enthaltende pharmazeutische Zusammensetzungen
O-arylmethyl N-(thio)acyl hydroxylamines as ligands for the melatonine receptors, their processes of preparation and the pharmaceutical compositions containing them

(30) Priorité: 26.10.1994 FR 9412784
(43) Date de publication de la demande: 01.05.1996
(73) Titulaire: ADIR ET COMPAGNIE, 92415 Courbevoie Cédex (FR)
(72) Inventeur: Andrieux, Jean, F-92160 Antony (FR); Langlois, Michel, F-92330 Sceaux (FR); Renard, Pierre, F-78000 Versailles (FR); Delagrange, Philippe, F-92130 Issy-Les-Moulineaux (FR)

(56) Documents cités:
- EP-A- 0 447 285
- EP-A- 0 527 687
- EP-A- 0 591 057

## Description

La présente invention concerne de nouvelles O-arylméthyl N-(thio)acyl hydroxylamines, leurs procédés de préparation, et les compositions pharmaceutiques qui les contiennent.

On connaît dans l'art antérieur des ligands mélatoninergiques à structures arylalkylamides (ou urées correspondantes). Ainsi, les demandes EP 0447285 et EP 0591057 décrivent des alkylamides naphtaléniques. On trouve également la demande EP 0527687 qui décrit des structures analogues en série hétérocyclique.

La demanderesse a découvert de nouvelles O-arylméthyl N-(thio)acyl hydroxylamines à structure originale, qui sont de puissants ligands des récepteurs de la mélatonine.

Outre leur action bénéfique sur les troubles du rythme circadien (J. Neurosurg. 1985, 63 : pp 321-341) et du sommeil (Psychopharmacology 1990, 100 : pp 222-226), les composés agissant sur le système mélatoninergique possèdent d'intéressantes propriétés pharmacologiques sur le système nerveux central, notamment anxiolytiques et antipsychotiques (Neuropharmacology of Pineal Secretions 1990, 8 (3-4): pp 264-272), analgésiques (Pharmacopsychiat. 1987, 20 pp 222-223), pour le traitement de la maladie de Parkinson (J. Neurosurg 1985, 63 : pp 321-341) et d'Alzheimer (Bran Research 1990, 528 : pp 170-174). De même, ces composés ont montré une activité sur certains cancers (Melatonin - Clinical Perspectives, Oxford University Press, 1988 : pp 164-165), sur l'ovulation (Science 1987, 227 : pp 714-720) et le diabète (Clinical endocrinology 1986, 24 : pp 359-364).

Plus particulièrement, l'invention concerne les composés de formule (I) : 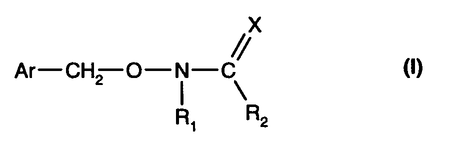 dans laquelle :
- X représente un atome d'oxygène ou de soufre,
- R₁ représente un atome d'hydrogène ou un radical choisi parmi alkyle, cycloalkyle, cycloalkylalkyle, aryle et arylalkyle,
- R₂ représente :
   - un radical R₂₁ qui est un hydrogène ou un radical choisi parmi :
      . un groupement alkyle non substitué ou substitué,
      . un groupement alcényle non substitué ou substitué,
      . un groupement alcynyle non substitué ou substitué,
      . et un groupement cycloalkyle ou cycloalkylalkyle non substitué ou substitué,
   - ou un radical R₂₂ choisi parmi :
      . un groupement alkylamino dans lequel le groupement alkyle peut être non substitué ou substitué
      . et un groupement cycloalkylamino ou cycloalkylalkylamino dans lequel le groupement cycloalkyle ou cycloalkylalkyle peut être non substitué ou substitué,
- Ar représente un groupement non substitué ou substitué choisi parmi :
   . naphtyle,
   . indolyle,
   . benzofuranyle,
   . benzothiophènyle,
   Ar pouvant également être partiellement ou totalement hydrogéné,
étant entendu que lors de la description de la formule (I) :
- les termes "alkyle" et "alkoxy" désignent des groupements linéaires ou ramifiés contenant de 1 à 6 atomes de carbone,
- les termes "alcényle" et "alcynyle" désignent des groupements insaturés linéaires ou ramifiés contenant de 2 à 6 atomes de carbone,
- le terme "cycloalkyle" désigne un groupement de 3 à 8 atomes de carbone,
- le terme "substitué" associé aux groupements alkyle, cycloalkyle, alcényle, alcynyle et cycloalkylalkyle signifie que la substitution peut se faire par un ou plusieurs radicaux choisis parmi halogène, alkyle et alkoxy,
- le terme "substitué" associé au groupement Ar signifie que Ar est substitué par un ou plusieurs radicaux R, identiques ou différents, choisi parmi halogène, hydroxyle, Ra, -CH₂-Ra, -O-Ra, -CO-Ra et -O-CO-Ra (avec Ra choisi parmi alkyl, alkyl substitué, alcényle, alcynyle, cycloalkyle, cycloalkyle substitué, cycloalkylalkyle, cycloalkylalkyle substitué, aryle, aryle substitué, arylalkyle et arylalkyle substitué),
- et le terme "substitué" associé aux expressions "aryle" et "arylalkyle" signifie que la substitution consiste en un ou plusieurs radicaux choisis parmi halogène, alkyle, alkoxy, hydroxy et alkyl substitué par un ou plusieurs halogènes ;
et leurs énantiomères ou diastéréoisomères.

De façon particulière, l'invention concerne les composés de formule (I) dans laquelle le groupement Ar est mono ou disubstitué selon l'une des formules suivantes : 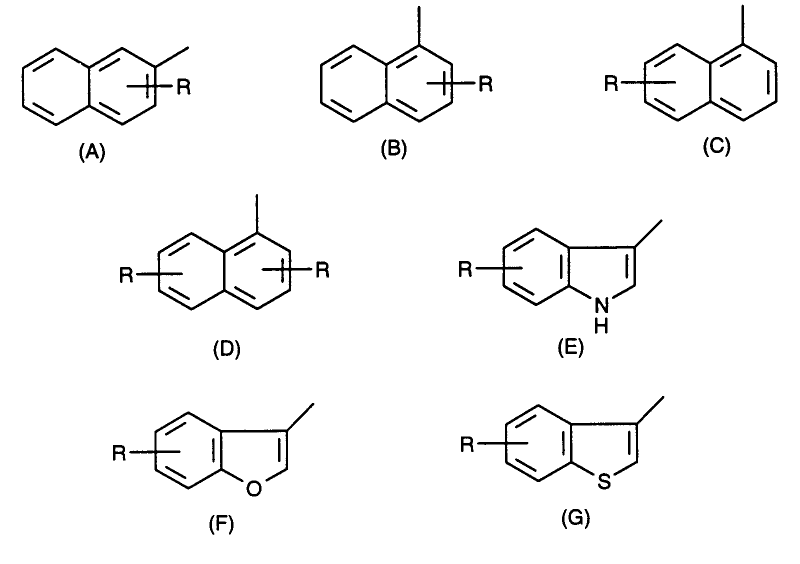 avec R tel que défini dans la formule (I).

De façon particulière, l'invention concerne les composés de formule (I) dans laquelle le groupement Ar est mono ou disubstitué selon l'une des formules suivantes: 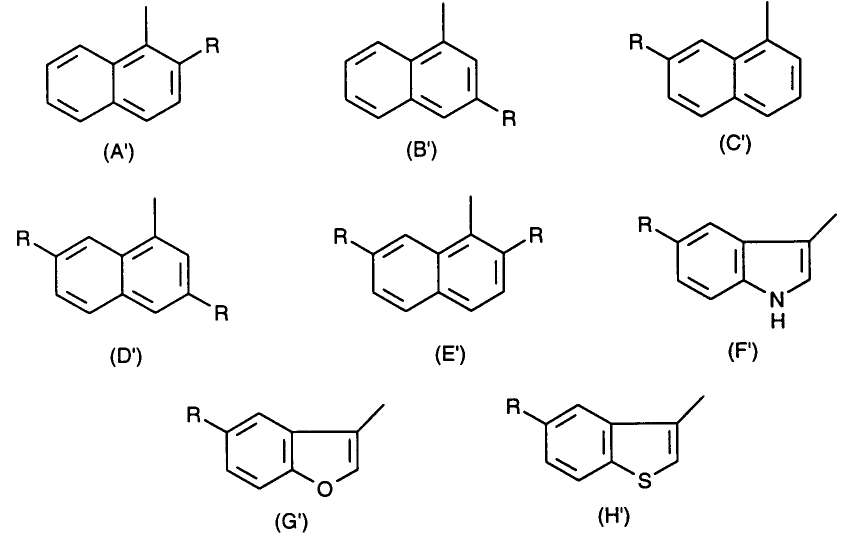 avec R tel que défini dans la formule (I).

Par exemple, l'invention concerne les composés suivants :
- O-[(7-méthoxy napht-1-yl)méthyl]N-acétyl hydroxylamine
- O-[(7-méthoxy napht-1-yl)méthyl]N-propionyl hydroxylamine
- O-[(7-méthoxy napht-1-yl)méthyl]N-cyclopropylcarbonyl hydroxylamine
- O-[(2-méthoxy napht-1-yl)méthyl]N-acétyl hydroxylamine
- O-[(2-méthoxy napht-1-yl)méthyl]N-propionyl hydroxylamine
- O-[(2-méthoxy napht-1-yl)méthyl]N-cyclopropylcarbonyl hydroxylamine
- O-[(napht-1-yl)méthyl]N-acétyl hydroxylamine

L'invention s'étend également au procédé de préparation des composés de formule (I), dans lequel on fait réagir un composé de formule (II) : 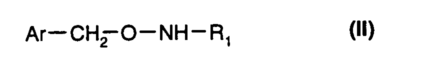 dans laquelle Ar et R₁ sont tels que définis dans la formule (I) avec un composé de formule (III) : 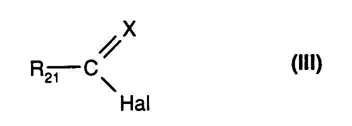 dans laquelle R₂₁ et X sont tels que définis dans la formule (I) et Hal représente un atome d'halogène, ou par l'aNhydride d'acide correspondant, ou avec de l'acide formique, pour obtenir un composé de 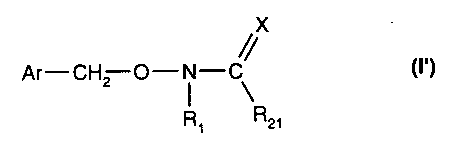 formule (I') : dans laquelle Ar, X, R₁ et R₂₁ sont tels que définis précédemment,
ou avec un composé de formule (III') : 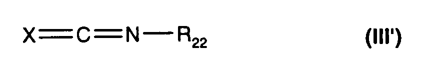 dans laquelle X et R₂₂ sont tels que définis dans la formule (I) pour obtenir un composé de formule (I'') : 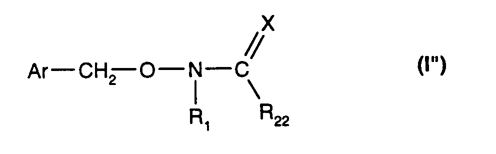 dans laquelle Ar, R₁, R₂₂ et X sont tels que définis précédemment,
les composés de formule (I') et (I'') formant l'ensemble des composés de formule (I), composés de formule (I) qui sont, le cas échéant :
- purifiés suivant une ou plusieurs méthodes de purification choisies parmi la cristallisation, la chromatographie, l'extraction, la filtration, et le passage sur charbon ou résine,
- ou séparés, le cas échéant, sous forme pure ou sous forme de mélange, en leurs éventuels énantiomères ou diastéréoisomères.

L'invention s'étend également au procédé de préparation des composés de formule (I/a) : 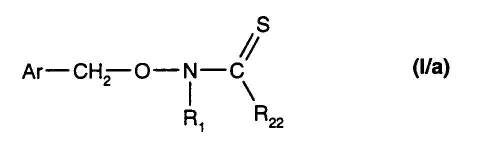 dans laquelle Ar, R₁ et R₂₂ sont tels que définis dans la formule (I), par réaction d'un composé de formule (I/b) : 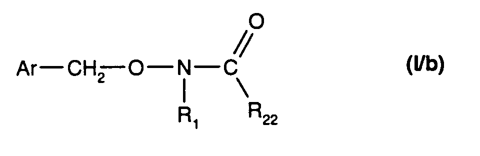 dans laquelle Ar, R₁ et R₂₂ sont tels que définis précédemment, avec un réactif de thionation, par exemple avec le réactif de Lawesson.

composés de formule (I/a) qui sont, le cas échéant :
- purifiés suivant une ou plusieurs méthodes de purification choisies parmi la cristallisation, la chromatographie, l'extraction, la filtration, et le passage sur charbon ou résine,
- ou séparés, le cas échéant, sous forme pure ou sous forme de mélange, en leurs éventuels énantiomères ou diastéréoisomères.

L'invention s'étend également au procédé de préparation des composés de formule (I/c) : 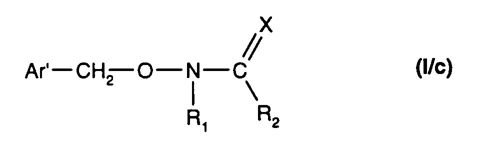 dans laquelle X, R₁ et R₂ sont tels que définis dans la formule (I) et Ar' représente un groupement de formule Ar tel que défini dans la formule (I) substitué par au moins un radical -CO-Ra, -O-CO-Ra, -OH et -CH₂-Ra,
caractérisé en ce que un composé de formule (I/d) : 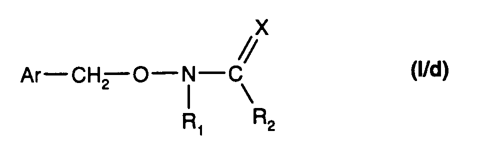 dans laquelle Ar est tel que défini dans la formule (I) et X, R₁ et R₂ sont tels que définis précédemment est soumis à une réaction de substitution avec un composé de formule (IV) : 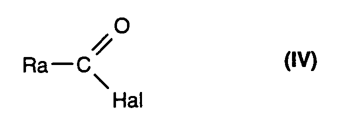 avec Ra tel que défini dans la formule (I) et Hal représentant un halogène, pour obtenir le composé de formule (I/e) correspondant : 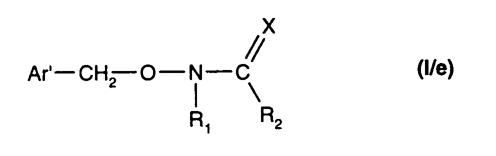 dans laquelle Ar', R₁, R₂ et X sont tels que définis précédemment, correspondant à un composé de formule (I/c) dans laquelle Ar' est substitué par au moins un substituant de formule -CO-Ra,
composé de formule (I/e) qui est :
- soit soumis à une oxydation par une réaction de Bayer-Villiger, afin d'oxyder le substituant -CO-Ra et obtenir un composé de formule (I/c) dans laquelle Ar' est substitué par au moins un substituant de formule -O-CO-Ra,
- soit soumis à une saponification pour obtenir un composé de formule (I/c) dans laquelle Ar' est substitué par au moins un substituant -OH,
- soit soumis à une réduction par le mercure et le zinc, en présence de toluène et d'acide chlorhydrique afin de réduire le substituant -CO-Ra en -CH₂-Ra, pour obtenir un composé de formule (I/c) dans laquelle Ar' est substitué par au moins un substituant -CH₂-Ra.

composés de formule (I/c) qui sont, le cas échéant :
- purifiés suivant une ou plusieurs méthodes de purification choisies parmi la cristallisation, la chromatographie, l'extraction, la filtration, et le passage sur charbon ou résine,
- ou séparés, le cas échéant, sous forme pure ou sous forme de mélange, en leurs éventuels énantiomères ou diastéréoisomères.

Les composés de formule (I) peuvent également être soumis si le groupement Ar présente un substituant -OCH₃, à une réaction de déméthylation par le tribromure de bore, afin d'obtenir la fonction hydroxyle correspondante.

Les matières premières utilisées dans le procédé précédemment décrit sont soit commerciales, soit aisément accessibles à l'homme du métier selon des procédés connus dans la littérature, soit accessibles par les préparations décrites ci-dessous.

Les composés de formule (I) possèdent des propriétés pharmacologiques très avantageuses.

Les composés de l'invention sont utiles dans la prévention et le traitement des troubles du système mélatoninergique.

L'étude pharmacologique des dérivés de l'invention a en effet montré qu'ils n'étaient pas toxiques, qu'ils étaient doués d'une très haute affinité sélective pour les récepteurs de la mélatonine et qu'ils présentent d'importantes activités sur le système nerveux central et en particulier, on a relevé des propriétés sur les troubles du sommeil, des propriétés anxiolytiques, antipsychotiques, analgésiques ainsi qu'une activité sur la circulation qui permettent d'établir que les produits de l'invention sont notamment utiles dans le traitement du stress, des troubles du sommeil, de l'anxiété, des dépressions saisonnières, des insomnies et fatigues dues aux décalages horaires, de la schizophrénie, des attaques de panique, de la mélancolie, de la régulation de l'appétit, de l'insomnie, des troubles psychotiques, de l'épilepsie, de la maladie de Parkinson, de la démence sénile, des divers désordres liés au vieillissement normal ou pathologique, de la migraine, des troubles vasculaires, des pertes de mémoire, de la maladie d'Alzheimer, ainsi que les troubles de la circulation cérébrale. Dans un autre domaine d'activité, il apparaît que les produits de l'invention possèdent des propriétés inhibitrices sur l'ovulation, d'immunomodulation et qu'ils sont susceptibles d'être utilisés dans le traitement de certains cancers hormonodépendants.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention, mais ne la limitent en aucune façon.

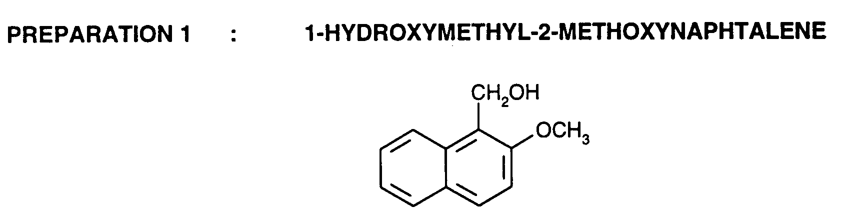

Dans un ballon d'un litre surmonté d'un réfrigérant ascendant, on met en suspension 0,11 mole d'aluminohydrure de lithium (AlLiH₄) dans 400 cm³ de tétrahydrofurane (THF) anhydre.

Sous vive agitation, on ajoute lentement 0,1 mole de 2-méthoxy-1-naphtaldéhyde commercial.
Le mélange est ensuite porté à 40°C pendant 30 min puis refroidi par un bain de glace ; on ajoute lentement un mélange de tétrahydrofurane et d'eau (90/10) de façon à détruire le complexe et l'alumino hydrure de lithium en excès.

Après filtration du précipité blanc minéral, la phase organique est lavée à l'eau puis séchée. Le solvant est éliminé sous vide ; on obtient ainsi l'alcool désiré pratiquement pur.
- Point de fusion :: 101-102°C
- Solvant de recristallisation :: éther-cyclohexane
- RMN (CDCl₃) :: 2,15 ppm (singulet, 1H, (-OH))
3,90 ppm (singulet, 3H, (-O-CH₃))
5,10 ppm (singulet, 2H, (Ar-CH₂-O))
7,20 à 8,20 ppm (massif, 6H, H aromatiques))

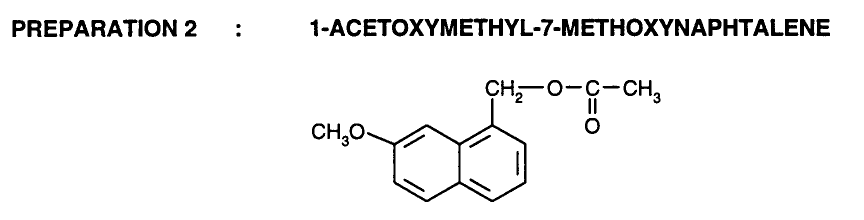

### Stade A : (7-méthoxy-1,2,3,4-tétrahydronaphtylidèn-1-yl)acétate d'éthyle

Mélanger 50 g de 7-méthoxy-1-tétralone, 40 g de bromoacétate d'éthyle et 150 cm³ de benzène par l'intermédiaire d'une ampoule à brome. Ajouter le mélange sur le zinc en aiguilles activé (18,6 g) selon Reformatsky et un cristal d'iode. Chauffer à 60°C et ensuite porter à reflux pendant 45 min. Hydrolyser sur de la glace en présence d'acide chlorydrique. Extraire au benzène, sécher et porter à ébullition en présence de P₂0₅. Filtrer et porter à sec.

Le résidu est utilisé tel quel dans l'étape suivante.
- Rendement :: 80 %

### Stade B : (7-méthoxy-napht-1-yl)acétate d'éthyle

Mélanger 50 g de (7-méthoxy-1,2,3,4-tétrahydronaphtylidène-1-yl)acétate d'éthyle à 7,35 g de soufre et chauffer à 215°C pendant 10 heures. Refroidir, ajouter 300 cm³ d'acétate d'éthyle, agiter pendant 30 min, filtrer. Porter à sec.

Le résidu obtenu est utilisé tel quel pour l'étape de saponification.

### Stade C : acide (7-méthoxy-napht-1-yl)acétique

Chauffer à reflux pendant 3 heures un mélange de (7-méthoxy-napht-1-yl)acétate d'éthyle obtenu précédemment dans 250 cm³ de soude à 20 % dans l'éthanol. Porter à sec et laver le résidu à l'éther. Précipiter par un courant d'acide chlorydrique gazeux.
- Point de fusion :: 155-156°C
- Rendement :: 68 %

### Stade D : 1-acétoxyméthyl-7-méthoxynaphtalène

0,1 mole d'acide (7-méthoxy-napht-1-yl)acétique obtenu précédemment sont dissous dans 200 cm³ de benzène anhydre et 100 cm³ d'acide acétique anhydre.

On porte le mélange à ébullition sous un courant d'Argon pour éliminer complétement les traces d'oxygène dissoutes dans le mélange réactionnel.

0,1 mole de tétracétate de plomb est ajouté à froid puis on chauffe lentement sous agitation et courant d'argon le mélange réactionne très foncé. Vers 70°C, un dégagement important de CO₂ se produit accompagné d'une décoloration de la solution. Les solvants sont alors éliminés sous pression réduite. Le résidu est repris par du dichlorométhane et de l'éther; le précipité minéral formé est filtré sur celite. Le filtrat est lavé successivement 2 fois par une solution de bicarbonate de sodium à 5 % puis à l'eau. La phase organique, séchée sur Na₂SO₄, est filtrée. Les solvants sont chassés sous vide conduisant à une huile crème constituée du composé désiré pratiquement pur. Cette huile peut être purifiée par chromatographie sur silice (éluant CH₂Cl₂).

L'acidification par HCl concentré des eaux de lavage au bicarbonate de sodium permet de récupérer l'acide de départ n'ayant pas réagi (10 %).
- Rendement :: 85 %
- RMN (CDCl₃) :: 2,10 ppm (singulet, 3H, 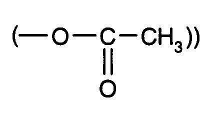 3,90 ppm (singulet, 3H, (-O-CH₃))
5,50 ppm (singulet, 2H, (Ar-CH₂-O-))
7,10 à 7,90 ppm (massif, 6H, (H aromatiques))

### PREPARATION 3 : 1-HYDROXYMETHYL-7-METHOXYNAPHTALENE

0,1 mole de 1-acétoxyméthyl-7-méthoxynaphthalène précédent est saponifié à température ambiante sous argon par une solution de 0,2 mole de soude diluée dans 200 cm³ de méthanol et 40 cm³ d'eau.

Le méthanol est éliminé sous vide puis le résidu est jeté sur de la glace et acidifié par de l'acide chlorydrique concentré. Le résidu est extrait à l'éther. Après lavage de la phase éthérée deux fois par l'eau, séchage sur Na₂SO₄, on élimine l'éther sous pression réduite conduisant à une huile cristallisant aussitôt par refroidissement.

L'alcool désiré est obtenu à l'état pratiquement pur, fondant après recristallisation.
- Point de fusion :: 71°C
- RMN (CDCl₃) :: 2,50 ppm (singulet élargi 1H, (-OH))
3,90 ppm (singulet, 3H, (-O-CH₃))
5,05 ppm (singulet, 2H, (Ar-CH₂-O))
7,10 à 7,85 (massif, 6H, (H aromatiques))

### PREPARATION 4 : 1-CHLOROMETHYL-2-METHOXYNAPHTALENE

0,1 mole de 1-hydroxyméthyl-2-méthoxynaphtalène (préparation 1) et 0,11 mole de pyridine sont dissous dans 100 cm³ de benzène anhydre.

Le mélange est refroidi à 0°C puis on ajoute sous agitation 0,11 mole de chlorure de thionyle dilué dans 50 cm³ de benzène anhydre.

Au bout d'une heure à température ambiante le mélange réactionnel est jeté sur de la glace puis extrait deux fois à l'éther. Les phases organiques réunies sont lavées successivement par une solution glacée de bicarbonate de sodium puis à l'eau, séchées sur sulfate de sodium et filtrées. Le filtrat est concentré sous pression réduite conduisant au dérivé chlorométhylé suffisamment pur pour être utilisé tel quel.

### PREPARATION 5 : 1-CHLOROMETHYL-7-METHOXYNAPHTALENE

En procédant de la même façon que pour la synthèse du composé de la préparation 4, mais en remplaçant le 1-hydroxyméthyl-2-méthoxynaphtalène par le 1-hydroxyméthyl-7-méthoxynaphtalène (préparation 3), on obtient le composé du titre.

### PREPARATION 6 : O-[2-METHOXYNAPHT-1-YL)METHYL]HYDROXYLAMINE

(D'après Kasztreiner E. et al. Acta. Chim. Acad. Sci. Hung. 1975, 84 (2), pp 167-180 et Daudon M. et al Bull. Soc. Chim. Fr. 1976, pp 833).

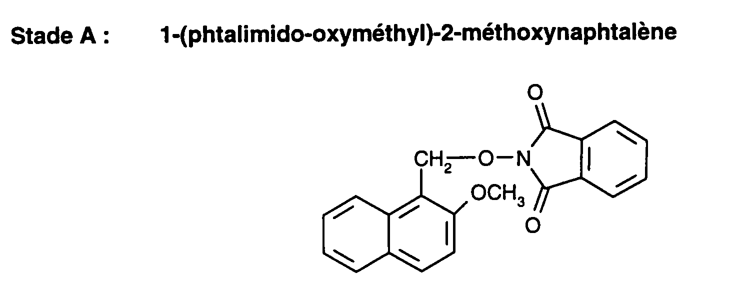

0,1 mole de N-hydroxyphtalimide est dissoute dans 60 cm³ de diméthylsulfoxide (DMSO) puis on ajoute sous agitation 0,1 mole d'acétate de sodium.

La solution devient rouge foncé. 0,1 mole de 1-chlorométhyl-2-méthoxy naphtalène (préparation 4) mis en solution dans 20 cm³ de DMSO est ajouté en une seule fois et le mélange réactionnel est chauffé jusqu'à décoloration (vers 100°C) puis refroidi. 500 cm³ de chloroforme sont ajoutés et la phase organique est lavée deux fois par une solution aqueuse saturée de NaCl.

La phase organique séchée sur sulfate de sodium est concentrée sous pression réduite pour éliminer le chloroforme et le DMSO.

Le résidu gommeux est repris par de l'éther ; le composé désiré est obtenu sous forme de précipité blanc qui est chromatographié sur silice (éluant CH₂Cl₂) pour éliminer les traces de N-hydroxyphtalimide de départ.
- Point de fusion :: 174°C
- Rendement :: 80 %
- RMN (CDCl₃) :: 3,90 ppm (singulet, 3H, (-O-CH₃))
5,80 ppm (singulet, 2H, (Ar-CH₂-O-))
7,20 à 8,50 ppm (massif, 10 H, (H aromatiques))

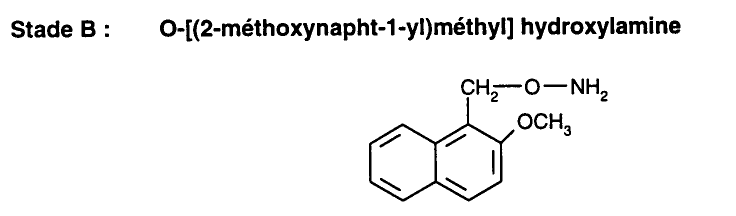

0,01 mole de 1-(phtalimido-oxyméthyl)-2-méthoxynaphtalène préparé au stade précédent et 30 cm³ d'éthanol sont placés dans un ballon surmonté d'un réfrigérant puis porté à ébullition ; on ajoute alors en une seule fois 0,02 mole d'hydrate d'hydrazine en solution dans 10 cm³ d'éthanol. Un précipité cotonneux de phtalhydrazide se forme presque aussitôt. On laisse poursuivre l'ébullition pendant 5 min supplémentaire puis, après refroidissement, le précipité est filtré.

La solution alcoolique est concentré sous pression réduite. Le résidu gommeux comprenant en majorité le composé du titre est repris par 20 cm³ de dichlorométhane et filtré. Le filtrat est concentré sous vide pour éliminer le solvant : un résidu huileux est obtenu. L'examen du spectre de résonance magnétique nucléaire (RMN) montre que le composé ne nécessite pas de purification supplémentaire.
- RMN (CDCl₃) :: 3,90 ppm (singulet, 3H, (-O-CH₃))
5,25 ppm (singulet, 2H, (aromatique -CH₂-O-))
5,30 ppm (singulet, 2H, (-NH₂))
7,05 à 8,20 ppm (massif, 6H, (H aromatiques))

### PREPARATION 7 : O-[(7-METHOXY NAPHT-1-YL)METHYL]HYDROXYLAMINE

En procédant de la même façon que pour la synthèse du composé de la préparation 6, mais en remplaçant au stade A le 1-chlorométhyl-2-méthoxy naphtalène par le 1-chlorométhyl-7-méthoxy naphtalène (préparation 5), on obtient le composé du titre.

### Stade A : 1-(phtalimido-oxyméthyl) 7-méthoxynaphtalène

Cristaux blancs
- point de fusion :: 202°C
- Rendement :: 90 %
- RMN (CDCl₃) :: 4,05 ppm (singulet, 3H, (-OCH₃))
5,50 ppm (singulet, 2H, (Ar-CH₂-O))
7,10 à 8,10 ppm (massif, 10H, (H aromatiques))

### Stade B : O-[(7-méthoxynapht-1-yl)méthyl]hydroxylamine

- RMN (CDCl₃) :: 1,85 ppm (singulet, 3H, (-COCH₃))
3,95 ppm (singulet, 3H, (-OCH₃))
5,30 ppm (singulet, 2H, (Ar-CH₂-O))
7,10 à 7,80 ppm (massif, 6H, (H aromatiques))
8,45 ppm (singulet, 1H, (NH))

### PREPARATION 8 : O-[(5-METHOXYBENZOTHIOPHEN-3-YL)METHYL]HYDROXYLAMINE

En procédant de la même façon que pour la synthèse des composés des préparations 2 (stade D), 3, 5 et 7, mais en remplaçant le produit de départ, c'est-à-dire l'acide (7-méthoxynapht-1-yl) acétique par l'acide (5-méthoxybenzothiophen-3-yl) acétique (J. Med. Chem. 1970, 13 (6), pp 1205-1208), on obtient le composé du titre.

### PREPARATION 9 : O-[(5-METHOXYBENZOFURAN-3-YL)METHYL]HYDROXYLAMINE

En procédant de la même façon que pour la synthèse des composés des préparations 2 (stade D), 3, 5 et 7, mais en remplaçant le produit de départ, c'est-à-dire l'acide (7-méthoxy-napht-1-yl)acétique par l'acide (5-méthoxy(benzofuran-3-yl)acétique (Zh. Org. Khim. 1967, 3(12), pp 2185-2188), on obtient le composé du titre.

### PREPARATION 10 : O-[(5-METHOXYINDOL-3-YL)METHYL]HYDROXYLAMINE

En procédant de la même façon que pour la synthèse des composés des préparations 2 (stade D), 3, 5 et 7, mais en remplaçant le produit de départ, c'est-à-dire l'acide (7-méthoxynapht-1-yl)acétique par l'acide (5-méthoxyindol-3-yl)acétique, on obtient le composé du titre.

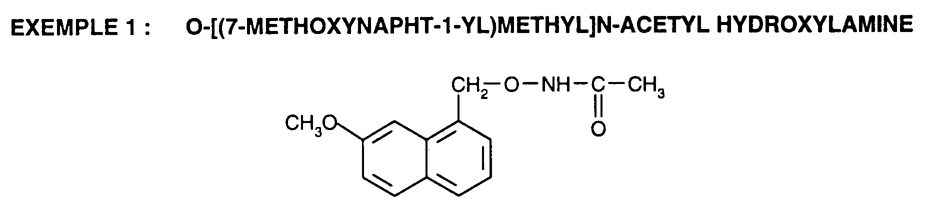

0,01 mole de O-[(7-méthoxynapht-1-yl)méthyl]hydroxylamine obtenue de la préparation 7 est dissoute dans 50 cm³ de chloroforme ; on ajoute 30 cm³ d'eau et 0,02 mole de K₂CO₃. Sous forte agitation et à 0°C on ajoute alors 0,012 mole de chlorure d'acide acétique (ou d'anhydride acétique). Au bout de 15 min à 0°C, 10 cm³ d'une solution aqueuse de NH₄OH est ajoutée (afin de détruire l'excès de chlorure d'acide ou d'anhydride acétiqe), puis 100 cm ³ d'eau supplémentaire. La phase organique est décantée, lavée deux fois avec de l'eau, puis séchée sur sulfate de sodium. Le solvant est évaporé sous pression réduite. Le résidu constitué du composé du titre est recristallisé dans de l'éther/dichlorométhane. On obtient ainsi des cristaux incolores.
- Point de fusion :: 125°C
- Rendement :: 90 %
- Solvant de recristallisation :: dichlorométhane-éther
- RMN (DCCl₃) :: 1,85 ppm (singulet, 3H, (-CO-CH₃))
3,95 ppm (singulet, 3H, (-O-CH₃))
5,30 ppm (singulet, 2H, (Ar-CH₂-O-))
7,10 à 7,80 ppm (massif, 6H, (H aromatiques))
8,45 ppm (singulet, 1H, (NH))

### EXEMPLE 2 : O-[(7-METHOXYNAPHT-1-YL)METHYL]N-PROPIONYL HYDROXYLAMINE

En procédant de la même façon que pour la synthèse du composé de l'exemple 1, mais en remplaçant lors de l'acylation le chlorure d'acide acétique par le chlorure d'acide propionique on obtient le composé du titre

### EXEMPLE 3 : O-[(7-METHOXYNAPHT-1-YL)METHYL]N-BUTYRYL HYDROXYLAMINE

En procédant de la même façon que pour la synthèse du composé de l'exemple 1, mais en remplaçant lors de l'acylation le chlorure d'acide acétique par le chlorure d'acide butyrique, on obtient le composé du titre sous forme de cristaux blancs.
- Point de fusion :: 98 °C
- Solvant de recristallisation :: éther/cyclohexane
- RMN (DCCl₃) :: 0,95 ppm (triplet (J=7Hz), 3H, (-CH₂-CH₂-CH₃))
1,75 ppm (multiplet mal résolu, 2H, (-CH₂-CH₂-CH₃))
2,10 ppm (triplet mal résolu, 2H, (-CH₂-CH₂-CH₃))
4,00 ppm (singulet, 3H, (-O-CH₃))
7,15 à 7,85 ppm (massif, 6H, (H aromatiques))
8,20 ppm (singulet, 1H, (NH)

### EXEMPLE 4 : O-[(7-METHOXYNAPHT-1-YL)METHYL)N-CROTONYL HYDROXYLAMINE

En procédant de la même façon que pour la synthèse du composé de l'exemple 1, mais en remplaçant lors de l'acylation le chlorure d'acide acétique par le chlorure d'acide crotonique, on obtient le composé du titre.

### EXEMPLE 5 : O-[(7-METHOXYNAPHT-1-YL)METHYL]N-IODACETYL HYDROXYLAMINE

En procédant de la même façon que pour la synthèse du composé de l'exemple 1, mais en remplaçant lors de l'acylation le chlorure d'acide acétique par le chlorure d'acide iodoacétique, on obtient le composé du titre.

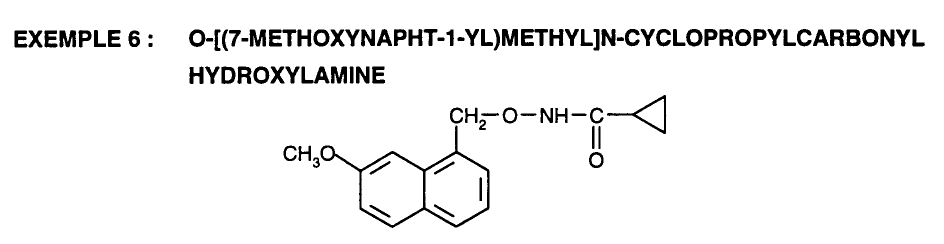

En procédant de la même façon que pour la synthèse du composé de l'exemple 1, mais en remplaçant lors de l'acylation le chlorure d'acide acétique par le chlorure d'acide cyclopropane carboxylique on obtient le composé du titre.
- Point de fusion :: 126°C
- Solvant de recristallisation :: cyclohexane
Cristaux incolores
- RMN (CDCl₃) :: 0,65 à 1,20 ppm (massif complexe, 5H, (H cyclopropaniques))
3,95 ppm (singulet, 3H, (O-CH₃))
5,30 ppm (singulet, 2H, (Ar-CH₂-O-))
7,15 à 7,80 ppm (massif, 5H, (H aromatiques))
8,40 ppm (singulet 1H, (-NH))

### EXEMPLE 7 : O-[(2-METHOXYNAPHT-1-YL)METHYL]N-ACETYL HYDROXYLAMINE

En procédant de la même façon que pour la synthèse du composé de l'exemple 1, mais en utilisant la O-[(2-méthoxynapht-1-yl)méthyl]hydroxylamine de la préparation 6, on obtient le composé du titre.
- Point de fusion :: 112°C
- Solvant de recristallisation :: éther/dichlorométhane
Cristaux incolores
- RMN (CDCl₃) :: 1,9 ppm (singulet, 3H, (-CO-CH₃))
3,95 ppm (singulet, 3H, (-O-CH₃))
5,45 ppm (singulet, 2H, (Ar-CH₂-O))
7,10 à 8,40 ppm (massif, 6H, (H aromatiques))
8,50 ppm (singulet 1H, (NH))

### EXEMPLE 8 : O-[(2-METHOXYNAPHT-1-YL)METHYL]N-PROPIONYL HYDROXYLAMINE

En procédant de la même façon que pour la synthèse du composé de l'exemple 7, mais en remplaçant dans l'étape d'acylation le chlorure d'acide acétique par le chlorure d'acide propionique, on obtient le composé du titre.
- Point de fusion :: 118°C
- Solvant de recristallisation :: éther
Cristaux incolores
- RMN (DCCl₃) :: 1,15 ppm (triplet mal résolu, 3H, (-CH₂-CH₃))
1,9 à 2,5 ppm (massif complexe, 2H, (-CH₂-CH₃))
3,90 ppm (singulet, 3H, (-O-CH₃))
5,50 ppm (singulet, 2H, (Ar-CH₂-O-))
7,10 à 8,40 ppm (massif, 6H, (H aromatiques))
8,80 ppm (singulet 1H, (NH))

### EXEMPLE 9 : O-[(2-METHOXYNAPHT-1-YL)METHYL]N-BUTYRYL HYDROXYLAMINE

En procédant de la même façon que pour la synthèse du composé de l'exemple 7, mais en remplaçant dans l'étape d'acylation le chlorure d'acide acétique par le chlorure d'acide butyrique, on obtient le composé du titre.

### EXEMPLE 10 : O-[(2-METHOXYNAPHT-1-YL)METHYL]N-CROTONYL HYDROXYLAMINE

En procédant de la même façon que pour la synthèse du composé de l'exemple 7, mais en remplaçant dans l'étape d'acylation le chlorure d'acide acétique par le chlorure d'acide crotonique, on obtient le composé du titre.

### EXEMPLE 11 : O-[(2-METHOXYNAPHT-1-YL)METHYL]N-IODOACETYL HYDROXYLAMINE

En procédant de la même façon que pour la synthèse du composé de l'exemple 7, mais en remplaçant dans l'étape d'acylation le chlorure d'acide acétique par le chlorure d'acide iodoacétique, on obtient le composé du titre.

### EXEMPLE 12 : O-[(2-METHOXYNAPHT-1-YL)METHYL]N-CYCLOPROPYLCARBONYL HYDROXYLAMINE

En procédant de la même façon que pour la synthèse du composé de l'exemple 7, mais en remplaçant dans l'étape d'acylation le chlorure d'acide acétique par le chlorure d'acide cyclopropane carboxylique, on obtient le composé du titre.
- Point de fusion :: 145 °C
- Solvant de recristallisation :: éther
Cristaux incolores
- RMN (CDCl₃) :: 0,51 à 1,30 ppm (multiplets, 5H, (H cyclopropaniques))
3,95 ppm (singulet, 3H, (-O-CH₃))
5,50 ppm (singulet, 2H, (Ar-CH₂-O-))
7,10 à 8,20 ppm (massif, 6H, (H aromatiques))
8,40 ppm (singulet 1H, (NH))

### EXEMPLE 13 : O-[(5-METHOXYINDOL-3-YL)METHYL]N-ACETYL HYDROXYLAMINE

En procédant de la même façon que pour la synthèse du composé de l'exemple 1, mais en remplaçant la O-[(7-méthoxynapht-1-yl)méthyl]hydroxylamine par la O-[5-méthoxyindol-3-yl)méthyl]hydroxylamine (préparation 10), on obtient le composé du titre.

### EXEMPLE 14 : O-[(5-METHOXYINDOL-3-YL)METHYL]N-PROPIONYL HYDROXYLAMINE

En procédant de la même façon que pour la synthèse du composé de l'exemple 13, mais en remplaçant dans l'étape d'acylation le chlorure d'acide acétique par le chlorure d'acide propionique, on obtient le composé du titre.

### EXEMPLE 15 : O-[(5-METHOXYINDOL-3-YL)]N-BUTYRYL HYDROXYLAMINE

En procédant de la même façon que pour la synthèse du composé de l'exemple 13, mais en remplaçant dans l'étape d'acylation le chlorure d'acide acétique par le chlorure d'acide butyrique, on obtient le composé du titre.

### EXEMPLE 16 : O-[(5-METHOXYINDOL-3-YL)METHYL]N-CROTONYL HYDROXYLAMINE

En procédant de la même façon que pour la synthèse du composé de l'exemple 13, mais en remplaçant le chlorure d'acide acétique par le chlorure d'acide crotonique, on obtient le composé du titre.

### EXEMPLE 17 : O-[(5-METHOXYINDOL-3-YL)METHYL]N-IODOACETYL HYDROXYLAMINE

En procédant de la même façon que pour la synthèse du composé de l'exemple 13, mais en remplaçant le chlorure d'acide acétique par le chlorure d'acide iodoacétique, on obtient le composé du titre.

### EXEMPLE 18 : O-[(5-METHOXYINDOL-3-YL)METHYL]N-CYCLOPROPYLCARBONYL HYDROXYLAMINE

En procédant de la même façon que pour la synthèse du composé de l'exemple 13, mais en remplaçant le chlorure d'acide acétique par le chlorure d'acide cyclopropane carboxylique, on obtient le composé du titre.

### EXEMPLE 19 : O-[(5-METHOXYBENZOTHIOPHEN-3-YL)METHYL]N-ACETYL HYDROXYLAMINE

En procédant de la même façon que pour l'exemple 1, mais en remplaçant la O-[(7-méthoxynapht-1 -yl)méthyl]hydroxylamine par la O-[(5-méthoxy benzothiophèn-3-yl)méthyl]hydroxylamine (préparation 8), on obtient le composé du titre.

### EXEMPLE 20 : O-[(5-METHOXYBENZOTHIOPHEN-3-YL)METHYL]N-PROPIONYL HYDROXYLAMINE

En procédant de la même façon que pour la synthèse du composé de l'exemple 19, mais en remplaçant dans l'étape d'acylation le chlorure d'acide acétique par le chlorure d'acide propionique, on obtient le composé du titre.

### EXEMPLE 21 : O-[(5-METHOXYBENZOFURAN-3-YL)METHYL]N-ACETYL HYDROXYLAMINE

En procédant de la même façon que pour la synthèse du composé de l'exemple 1, mais en remplaçant le O-[(7-méthoxynapht-1-yl)méthyl]hydroxylamine par le O-[(5-méthoxybenzofuran-3-yl)méthyl]hydroxylamine (préparation 9), on obtient le composé du titre

### EXEMPLE 22 : O-[(5-METHOXYBENZOFURAN-3-YL)METHYL]N-BUTYRYL HYDROXYLAMINE

En procédant de la même façon que pour la synthèse du composé de l'exemple 21, mais en remplaçant dans l'étape d'acylation le chlorure d'acide acétique par le chlorure d'acide butyrique, on obtient le composé du titre.

### EXEMPLE 23 : O-[(5-METHOXYBENZOFURAN-3-YL)METHYL]N-CYCLOPROPYLCARBONYL HYDROXYLAMINE

En procédant de la même façon que pour la synthèse du composé de l'exemple 21, mais en remplaçant dans l'étape d'acylation le chlorure d'acide acétique par le chlorure d'acide cyclopropane carboxylique, on obtient le composé du titre.

### EXEMPLES 24 A 50 :

En procédant de la même façon que pour la synthèse des composés précédents, mais en employant les réactifs appropriés, on obtient les composés des exemples 24 à 50.

Pour synthétiser les composés présentant une urée ou thiourée au niveau de l'azote de l'hydroxylamine, on procéde par réaction avec un isocyanate ou un isothiocyanate d'alkyle, de cycloalkyle ou d'alkylcycloalkyle tel que défini dans la formule (I) à une suspension des préparations 6 à 10.

### EXEMPLE 24 : O-[(5-METHOXYINDOL-3-YL)METHYL]N-TRIFLUOROACETYL HYDROXYLAMINE

### EXEMPLE 25 : N-[(5-METHOXYINDOL-3-YL)METHYLOXY]N'-PROPYLUREE

### EXEMPLE 26 : N-[(5-METHOXYINDOL-3-YL)METHYLOXY]N'-BUTYLUREE

### EXEMPLE 27 : O-[(7-METHOXYNAPHT-3-YL)METHYL]N-TRIFLUOROACETYL HYDROXYLAMINE

### EXEMPLE 28 : O-[(7-METHOXYNAPHT-1-YL)METHYL]N-CYCLOBUTYLCARBONYL HYDROXYLAMINE

### EXEMPLE 29 : N-[(7-METHOXYNAPHT-1-YL)METHYLOXY]N'-PROPYLUREE

### EXEMPLE 30 : N-[(7-METHOXYNAPHT-1-YL)METHYLOXY]N'-BUTYLUREE

### EXEMPLE 31 : N-[(7-METHOXYNAPHT-1-YL)METHYLOXY]N'-METHYLUREE

### EXEMPLE 32 : O-[(3-ACETYL-7-METHOXYNAPHT-1-YL)METHYL]N-ACETYL HYDROXYLAMINE

En partant d'une solution du composé de l'exemple 1 et de AlCl₃, dans un solvant tel que le nitrobenzène, on ajoute goutte à goutte le chlorure d'acétyle à 12°C et sous azote. On laisse agir pendant 2 heures à 12°C et on verse le mélange réactionnel sur la glace. On extrait, on sèche, on concentre et chromatographie le brut sur silice. On obtient le composé du titre.

### EXEMPLES 33 A 35 :

En procédant comme dans l'exemple 32 mais en utilisant les chlorures d'acyle appropriés, on obtient les composés des exemples suivants :

### EXEMPLE 33 : O-[(3-PROPIONYL-7-METHOXYNAPHT-1-YL)METHYL]N-ACETYL HYDROXYLAMINE

### EXEMPLE 34 : O-[(3-CYCLOPROPYLCARBONYL-7-METHOXYNAPHT-1-YL)METHYL] N-ACETYL HYDROXYLAMINE

### EXEMPLE 35 : O-[(3-BENZOYL-7-METHOXYNAPHT-1-YL)METHYL]N-ACETYL HYDROXYLAMINE

### EXEMPLE 36 : O-(3-ETHYL-7-METHOXYNAPHT-1-YL)N-ACETYL HYDROXYLAMINE

En partant d'une solution de 72 mg de chlorure mercurique dans 5,5 cm³ d'eau, on ajoute 3,6 g de zinc en poudre. On agite pendant 30 min. On laisse décanter et on élimine l'eau.

On ajoute alors 3,6 cm³ d'eau à cet amalgame, puis 3,6 cm³ d'acide chlorhydrique concentré, puis 5,26 mmoles du composé préparé selon l'exemple 32, et 25 cm³ de toluène.

On porte à reflux pendant 2h on extrait la phase organique, on lave à l'eau et après avoir séché sur sulfate de magnésium, on filtre et évapore à sec. On obtient ainsi le composé du titre.

### EXEMPLES 37 A 39 :

En procédant comme dans l'exemple 36 mais en partant des exemples 33 à 35, on obtient les composés des exemples suivants :

### EXEMPLE 37 : O-[(3-PROPYL-7-METHOXYNAPHT-1-YL)]N-ACETYL HYDROXYLAMINE

### EXEMPLE 38 : O-[(3-CYCLOPROPYLMETHYL-7-METHOXY)METHYL)]N-ACETYL HYDROXYLAMINE

### EXEMPLE 39 : O-[(3-BENZYL-7-METHOXYNAPHT-1-YL)METHYL]N-ACETYL HYDROXYLAMINE

### EXEMPLES 40 A 105

En procédant comme dans les exemples précédents mais en utilisant les réactifs appropriés, on obtient les composés des exemples suivants :

### EXEMPLE 40 : O-[(3-ETHYL-7-METHOXYNAPHT-1-YL]N-CYCLOPROPYLCARBONYL HYDROXYLAMINE

### EXEMPLE 41 : N-[(7-METHOXYNAPHT-1-YL)METHYLOXY]N'-PROPYLTHIOUREE

### EXEMPLE 42 : N-[(7-METHOXYNAPHT-1-YL)METHYLOXY]N'-BUTYLTHIOUREE

### EXEMPLE 43 : N-[(7-METHOXYNAPHT-1-YL)METHYLOXY]N'-METHYLTHIOUREE

### EXEMPLE 44 : N-[(5-METHOXYBENZOTHIOPHEN-3-YL)METHYLOXY]N'-METHYLTHIOUREE

### EXEMPLE 45 : O-[(5-METHOXYBENZOTHIOPHEN-3-YL)METHYL]N-THIOPROPIONYL HYDROXYLAMINE

### EXEMPLE 46 : N-[(5-METHOXYBENZOTHIOPHEN-3-YL)METHYLOXY]N'-PROPYLTHIOUREE

### EXEMPLE 47 : O-[(5-METHOXYBENZOFURAN-3-YL)METHYL]N-THIOACETYL HYDROXYLAMINE

### EXEMPLE 48 : N-[(5-METHOXYBENFURAN-3-YL)METHYLOXY]N'-METHYLTHIOUREE

### EXEMPLE 49 : N-[(5-METHOXYBENZOFURAN-3-YL)METHYLOXY]N'-PROPYLTHIOUREE

### EXEMPLE 50 : N-[(5-METHOXYBENZOFURAN-3-YL)METHYL]N'-CYCLOPROPYLTHIOUREE

### EXEMPLE 51 : O-[(3-ACETYLNAPHT-1-YL)METHYL]N-ACETYL HYDROXYLAMINE

### EXEMPLE 52 : O-[(3-CYCLOPROPIONYLNAPHT-1-YL)METHYL]N-ACETYL HYDROXYLAMINE

### EXEMPLE 53 : O-[(3-BENZOYLNAPHT-1-YL)METHYL]N-ACETYL HYDROXYLAMINE

### EXEMPLE 54 : O-[(3-BENZYLNAPHT-1-YL)METHYL]N-ACETYL HYDROXYLAMINE

### EXEMPLE 55 : O-[(3-ETHYLNAPHT-1-YL)METHYL]N-ACETYL HYDROXYLAMINE

### EXEMPLE 56 : O[(3-ACETOXY-7-METHOXYNAPHT-1-YL)METHYL]N-ACETYL HYDROXYLAMINE

A une solution de 9,81 mmol du composé de l'exemple 32 dans 125 cm³ de méthanol, est ajoutée une solution de 12,3 mmol de sel de magnésium de l'acide monoperoxyphtalique dans 100 cm³ d'eau ajustée à pH 5 avec NaOH 1N. Agiter à température ambiante pendant 24 heures. Evaporer le méthanol, ajouter NaHCO₃ 1N, extraire avec CH₂Cl₂, sécher sur MgSO₄, filtrer et concentrer.
On obtient le composé du titre.

### EXEMPLE 57 : O-[(3-HYDROXY-7-METHOXYNAPHT-1-YL)METHYL]N-ACETYL HYDROXYLAMINE

Dissoudre le résidu obtenu dans l'exemple 56 dans 200 cm³ de méthanol et traiter avec 250 cm³ de NaOH 0,05 N durant 1 heure à température ambiante. Evaporer le méthanol, amener à pH 12 avec NaOH 1N, extraire avec CH₂Cl₂, sécher sur MgSO₄, filtrer et concentrer. Chromatographier sur silice. On obtient le composé du titre.

### EXEMPLE 58 : O-[(7-ETHOXYNAPHT-1-YL)METHYL]N-ACETYL HYDROXYLAMINE

### EXEMPLE 59 : O-[(7-PROPOXYNAPHT-1-YL)METHYL]N-ACETYL HYDROXYLAMINE

### EXEMPLE 60: O-[(7-ETHYLNAPHT-1-YL)METHYL]N-ACETYL HYDROXYLAMINE

### EXEMPLE 61 : O-[(NAPHT-1-YL)METHYL]N-ACETYL HYDROXYLAMINE

### EXEMPLE 62 : O-[(7-ETHOXYNAPHT-1-YL)METHYL]N-CYCLOPROPYLCARBONYLE HYDROXYLAMINE

### EXEMPLE 63 : O-[(7-PROPOXYNAPHT-1-YL)METHYL]N-CYCLOPROPYLCARBONYLE HYDROXYLAMINE

### EXEMPLE 64 : O-[(7-ETHYLNAPHT-1-YL)METHYL]N-CYCLOPROPYLCARBONYLE HYDROXYLAMINE

### EXEMPLE 65 : O-[(NAPHT-1-YL)METHYL]N-CYCLOPROPYLCARBONYLE HYDROXYLAMINE

### EXEMPLE 66 : O-[(NAPHT-1-YL)METHYL]N-CYCLOBUTYLCARBONYLE HYDROXYLAMINE

### EXEMPLE 67 : O-[(2-ETHOXYNAPHT-1-YL)METHYL]N-ACETYL HYDROXYLAMINE

### EXEMPLE 68 : O-[(2-PROPOXYNAPHT-1-YL)METHYL]N-ACETYL HYDROXYLAMINE

### EXEMPLE 69 : O-[(2-ETHYLNAPHT-1-YL)METHYL]N-ACETYL HYDROXYLAMINE

### EXEMPLE 70 : O-[(2-ETHOXYNAPHT-1-YL)METHYL]N-CYCLOPROPYLCARBONYL HYDROXYLAMINE

### EXEMPLE 71 : O-[(2-PROPOXYNAPHT-1-YL)METHYL]N-CYCLOPROPYLCARBONYL HYDROXYLAMINE

### EXEMPLE 72 : O-[(2-ETHYLNAPHT-1-YL)METHYL]N-CYCLOPROPYLCARBONYL HYDROXYLAMINE

### EXEMPLE 73 : O-[(2-ETHYLNAPHT-1-YL)METHYL]N-CYCLOBUTYLCARBONYL HYDROXYLAMINE

### EXEMPLE 74 : O-[(5-ETHOXYINDOL-3-YL)METHYL]N-ACETYL HYDROXYLAMINE

### EXEMPLE 75 : O-[(5-PROPOXYINDOL-3-YL)METHYL]N-ACETYL HYDROXYLAMINE

### EXEMPLE 76 : O-[(5-ETHYLINDOL-3-YL)METHYL]N-ACETYL HYDROXYLAMINE

### EXEMPLE 77 : O-[(INDOL-3-YL)METHYL]N-ACETYL HYDROXYLAMINE

### EXEMPLE 78 : O-[(5-ETHOXYINDOL-3-YL)METHYL]N-CYCLOPROPYLCARBONYL HYDROXYLAMINE

### EXEMPLE 79 : O-[(5-PROPOXYINDOL-3-YL)METHYL]N-CYCLOPROPYLCARBONYL HYDROXYLAMINE

### EXEMPLE 80 : O-[(5-ETHYLINDOL-3-YL)METHYL]N-CYCLOPROPYLCARBONYL HYDROXYLAMINE

### EXEMPLE 81 : O-[(INDOL-3-YL)METHYL]N-CYCLOPROPYLCARBONYL HYDROXYLAMINE

### EXEMPLE 82 : O-[(INDOL-3-YL)METHYL]N-CYCLOBUTYLCARBONYL HYDROXYLAMINE

### EXEMPLE 83 : O-[(5-ETHOXYBENZOTHIOPHEN-3-YL)METHYL]N-ACETYL HYDROXYLAMINE

### EXEMPLE 84 : O-[(5-PROPOXYBENZOTHIOPHEN-3-YL)METHYL]N-ACETYL HYDROXYLAMINE

### EXEMPLE 85 : O-[(5-ETHYLBENZOTHIOPHEN-3-YL)METHYL]N-ACETYL HYDROXYLAMINE

### EXEMPLE 86 : O-[(BENZOTHIOPHEN-3-YL)METHYL]N-ACETYL HYDROXYLAMINE

### EXEMPLE 87 : O-[(5-ETHOXYBENZOTHIOPHEN-3-YL)METHYL]N-CYCLOPROPYLCARBONYL HYDROXYLAMINE

### EXEMPLE 88 : O-[(5-PROPOXYBENZOTHIOPHEN-3-YL)METHYL]N-CYCLOPROPYLCARBONYL HYDROXYLAMINE

### EXEMPLE 89 : O-[(5-ETHYLBENZOTHIOPHEN-3-YL)METHYL]N-CYCLOPROPYLCARBONYL HYDROXYLAMINE

### EXEMPLE 90 : O-[(BENZOTHIOPHEN-3-YL)METHYL]N-CYCLOPROPYLCARBONYL HYDROXYLAMINE

### EXEMPLE 91 : O-[(BENZOTHIOPHEN-3-YL)METHYL]N-CYCLOBUTYLCARBONYL HYDROXYLAMINE

### EXEMPLE 92 : O-[(5-ETHOXYBENZOFURAN-3-YL)METHYL]N-ACETYL HYDROXYLAMINE

### EXEMPLE 93 : O-[(5-PROPOXYBENZOFURAN-3-YL)METHYL]N-ACETYL HYDROXYLAMINE

### EXEMPLE 94 : O-[(5-ETHYLBENZOFURAN-3-YL)METHYL]N-ACETYL HYDROXYLAMINE

### EXEMPLE 95 : O-[(BENZOFURAN-3-YL)METHYL]N-ACETYL HYDROXYLAMINE

### EXEMPLE 96 : O-[(5-ETHOXYBENZOFURAN-3-YL)METHYL]N-CYCLOPROPYLCARBONYL HYDROXYLAMINE

### EXEMPLE 97 : O-[(5-PROPOXYBENZOFURAN-3-YL)METHYL]N-CYCLOPROPYLCARBONYL HYDROXYLAMINE

### EXEMPLE 98 : O-[(5-ETHYLBENZOFURAN-3-YL)METHYL]N-CYCLOPROPYLCARBONYL HYDROXYLAMINE

### EXEMPLE 99 : O-[(BENZOFURAN-3-YL)METHYL]N-CYCLOPROPYLCARBONYL HYDROXYLAMINE

### EXEMPLE 100 : O-[(BENZOFURAN-3-YL)METHYL]N-CYCLOBUTYLCARBONYL HYDROXYLAMINE

### EXEMPLE 101 : O-[(2,7-DIMETHOXYNAPHT-1-YL)METHYL]N-ACETYL HYDROXYLAMINE

### EXEMPLE 102 : O-[(2,7-DIMETHOXYNAPHT-1-YL)METHYL]N-CYCLOPROPYLCARBONYL HYDROXYLAMINE

### EXEMPLE 103 : O-[(2,7-DIMETHOXYNAPHT-1-YL)METHYL]N-TRIFLUOROACETYL HYDROXYLAMINE

### EXEMPLE 104 : O-[(2,7-DIMETHOXYNAPHT-1-YL)METHYL]N-PROPIONYL HYDROXYLAMINE

### EXEMPLE 105 : O-[(2,7-DIMETHOXYNAPHT-1-YL)METHYL]N-CYCLOBUTYLCARBONYL HYDROXYLAMINE

### ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La dose létale 50 (DL 50), entraînant la mort de 50 % des animaux, a été évaluée.

La DL 50 des produits testés est supérieure à 1000 mg.kg⁻¹ pour les composés étudiés ce qui indique la faible toxicité des composés de l'invention.

### EXEMPLE B : TEST DES QUATRE PLAQUES

Les produits de l'invention sont administrés par voie oesophagienne 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE C : ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO2/N2 5/95 %. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.
On a effectué le même type de mesure simultanément :
- sur le crémaster perfusé normalement,
- sur le crémaster sous ligature, c'est-à-dire le crémaster ischémié 2, 7, 14 et 21 jours après ligature.
Deux groupes d'animaux ont été étudiés :
- un groupe témoin sans traitement,
- un groupe traité per os par un produit de l'invention, à raison de 0,1 mg.kg⁻¹ par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitment de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

### EXEMPLE D : STIMULATION DES REPONSES IMMUNITAIRES

A des groupes de six souris on a administré des globules rouges de moutons. Ces groupes de souris ont ensuite été traités par voie sous cutanée par les composés de l'invention pendant six jours et un groupe témoin a été traité par un placebo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

### EXEMPLE E : INHIBITION DE L'OVULATION

On utilise des rates femelles adultes avec des cycles réguliers de quatre jours.

Des frottis vaginaux quotidiens ont été réalisés et des rates ont été sélectionnées après qu'elles ont montré au moins deux cycles consécutifs de quatre jours.

Chaque cycle est constitué de deux jours de dioestrus, un jour de proestrus et un jour d'oestrus.

L'après-midi du jour de proestrus, l'hormone lutéinisante est libére dans le sang par l'hypophyse. Cette hormone induit l'ovulation qui se traduit par la présence d'oeufs au niveau de l'oviducte le jour de l'oestrus.

Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées.

### EXEMPLE F : MISE EN EVIDENCE DE L'ACTIVITE ANALGESIQUE

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 1954, 119, 1874). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème min après l'injection.

Il est apparu que les composés de l'invention possèdent une activité analgésique.

### EXEMPLE G : ETUDE DE LIAISON AUX RECEPTEURS DE LA MELATONINE

### B1) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology 1989, 1, pp 1-4).

### PROTOCOLE

1) Les membranes de pars Tuberalis de mouton sont préparées et utilisées comme tissu cible dans des expériences de saturation pour déterminer les capacités et affinités de liaison pour la 2-[¹²⁵l] - iodomélatonine.
2) Les membranes de Pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

II apparaît que les composés de l'invention possèdent une puissante affinité pour les récepteurs de la mélatonine supérieure à celle de la mélatonine elle-même.

### B2) Etude sur des membranes de cellules du cerveau de poulet (gallus domesticus)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 1991, 128, pp 475-482). La [¹²⁵l] mélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman^{®} LS 6000.

Les produits utilisés sont :
- 2-[¹²⁵l] mélatonine
- mélatonine
- produits courants
- molécules originales

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correspondent aux valeurs de l'affinité montrent que la liaison des composés testés aux récepteurs mélatoninergiques est très puissante.

### EXEMPLE H : ETUDE DE L'ACTIVITE DES COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.
Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.
Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### RESULTATS

Il apparaît clairement que les composés de l'invention permettent d'agir de façon puissante sur le rythme circadien via le système mélatoninergique.

### EXEMPLE I : ACTIVITE ANTIARYTHMIQUE

### PROTOCOLE (Ref : LAWSON J.W. et al. J. Pharmacol. Expert. Therap. 1968, 160, pp 22-31)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE J : COMPOSITION PHARMACEUTIQUE : COMPRIMES

Comprimés dosés à 5 mg de O-[(7-méthoxynapht-1-yl)méthyl]N-butyryl hydroxylamine. formulation pour préparer 1000 comprimés.

| | |
|---|---|
| O-[(7-méthoxynapht-1-yl)méthyl]N-butyryl hydroxylamine | 5 g |
| Amidon de blé | 15 g |
| Amidon de maïs | 15 g |
| Stéarate de magnésium | 2 g |
| Silice | 1 g |
| Hydroxypropyl cellulose | 2 g |

## Revendications

1. Composés de formule (I) : 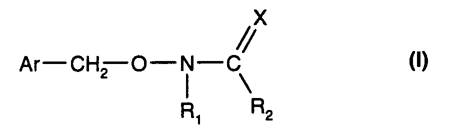 dans laquelle :
- X représente un atome d'oxygène ou de soufre,
- R₁ représente un atome d'hydrogène ou un radical choisi parmi alkyle, cycloalkyle, cycloalkylalkyle, aryle et arylalkyle,
- R₂ représente :
- un radical R₂₁ qui est un hydrogène ou un radical choisi parmi :
. un groupement alkyle non substitué ou substitué,
. un groupement alcényle non substitué ou substitué,
. un groupement alcynyle non substitué ou substitué,
. et un groupement cycloalkyle ou cycloalkylalkyle non substitué ou substitué,
- ou un radical R₂₂ choisi parmi :
. un groupement alkylamino dans lequel le groupement alkyle peut être non substitué ou substitué
. et un groupement cycloalkylamino ou cycloalkylalkylamino dans lequel le groupement cycloalkyle ou cycloalkylalkyle peut être non substitué ou substitué,
- Ar représente un groupement non substitué ou substitué choisi parmi
. naphtyle,
. indolyle,
. benzofuranyle,
. benzothiophènyle,
Ar pouvant également être partiellement ou totalement hydrogéné,
étant entendu que lors de la description de la formule (I) :
- les termes "alkyle" et alkoxy" désignent des groupements linéaires ou ramifiés contenant de 1 à 6 atomes de carbone,
- les termes "alcényle" et "alcynyle" désignent des groupements insaturés linéaires ou ramifiés contenant de 2 à 6 atomes de carbone,
- le terme "cycloalkyle" désigne un groupement de 3 à 8 atomes de carbone,
- le terme "substitué" associé aux groupements alkyle, cycloalkyle, alcényle, alcynyle et cycloalkylalkyle signifie que la substitution peut se faire par un ou plusieurs radicaux choisis parmi halogène, alkyle et alkoxy,
- le terme "substitué" associé au groupement Ar signifie que Ar est substitué par un ou plusieurs radicaux R, identiques ou différents, choisi parmi halogène, hydroxyle, Ra, -CH₂-Ra, -O-Ra, -CO-Ra et -O-CO-Ra (avec Ra choisi parmi alkyl, alkyl substitué, alcényle, alcynyle, cycloalkyle, cycloalkyle substitué, cycloalkylalkyle, cycloalkylalkyle substitué, aryle, aryle substitué, arylalkyle et arylalkyle substitué),
- et le terme "substitue" associé aux expressions "aryle" et "arylalkyle" signifie que la substitution consiste en un ou plusieurs radicaux choisis parmi halogène, alkyle, alkoxy, hydroxy et alkyl substitué par un ou plusieurs halogènes;
et leurs énantiomères ou diastéréoisomères.

2. Composés selon la revendication 1 dans laquelle le groupement Ar est mono ou disubstitué selon l'une des formules suivantes : 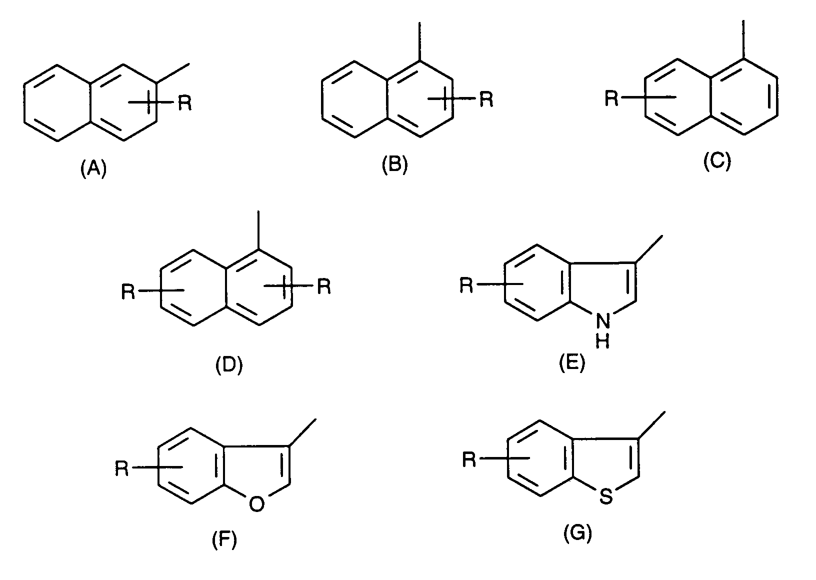 avec R tel que défini dans la revendication 1.

3. Composés selon la revendication 1 dans laquelle le groupement Ar est mono ou disubstitué selon l'une des formules suivantes : 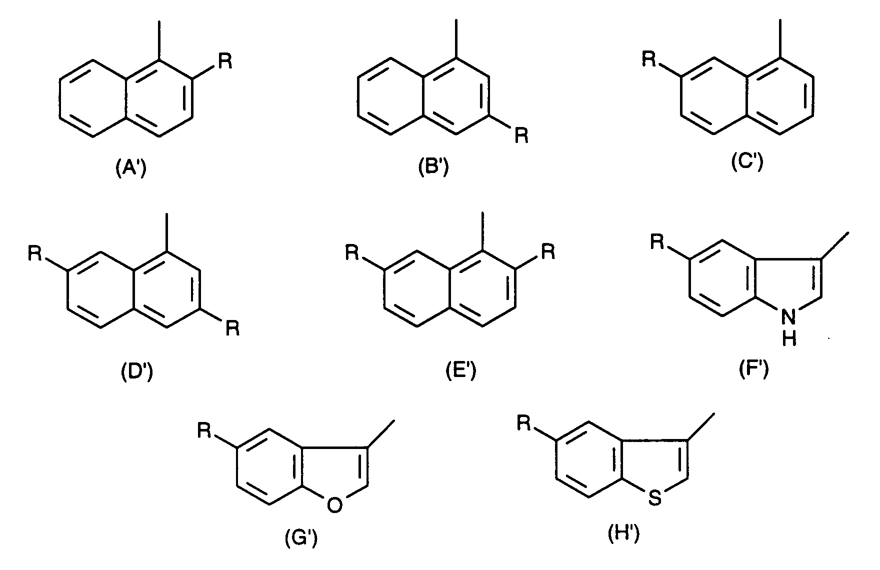 avec R tel que défini dans la formule (I).

4. Composé selon les revendication 1 à 3 qui est le O-[(7-méthoxynapht-1-yl)méthyl]N-acétyl hydroxylamine.

5. Composé selon les revendications 1 à 3 qui est le O-[(7-méthoxynapht-1-yl)méthyl]N-butyryl hydroxylamine.

6. Composé selon les revendications 1 à 3 qui est le O-[(7-méthoxynapht-1-yl)méthyl]N-cyclopropylcarbonyl hydroxylamine.

7. Composé selon les revendications 1 à 3 qui est le O-[(2-méthoxynapth-1-yl)méthyl]N-acétyl hydroxylamine.

8. Procédé de préparation des composés de formule (I), dans lequel on fait réagir un composé de formule (II) : 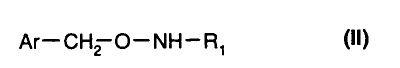 dans laquelle Ar et R₁ sont tels que définis dans la revendication 1 avec un composé de formule (III) : 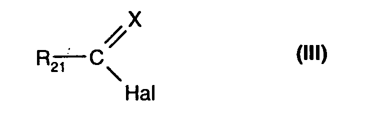 dans laquelle R₂₁ et X sont tels que définis dans la revendication 1 et Hal représente un atome d'halogène, ou par l'anhydride d'acide correspondant ou avec de l'acide formique, pour obtenir un composé de formule (I') : 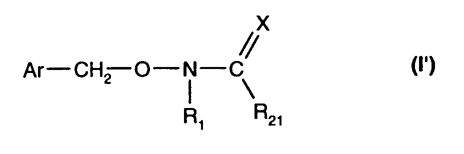 dans laquelle Ar, X, R₁ et R₂₁ sont tels que définis précédemment,
ou avec un composé de formule (III') : 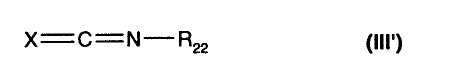 dans laquelle X et R₂₂ sont tels que définis dans la revendication 1 pour obtenir un composé de formule (I'') : 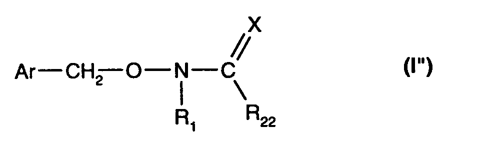 dans laquelle Ar, R₁ R₂₂ et X sont tels que définis précédemment,
l'ensemble des composés de formule (I') et (I'') formant les composés de formule générale (I) qui peuvent être purifiés selon une technique classique de séparation, que l'on transforme, si on le souhaite en leurs sels d'addition à un acide ou à une base pharmaceutiquement acceptable.

9. Compositions pharmaceutiques contenant au moins un composé de formule (I) selon l'une des revendications 1 à 7 en combinaison avec un ou plusieurs excipients pharmaceutiquement acceptables.

10. Compositions pharmaceutiques selon la revendication 9 utiles dans la prévention et le traitement des troubles du système mélatoninergique.

## Claims

1. Compounds of formula (I): 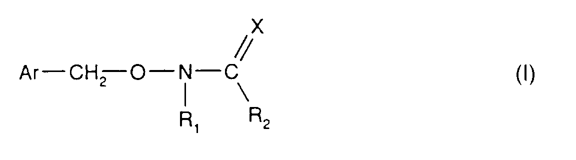 in which:
- X represents an oxygen atom or a sulphur atom;
- R₁ represents a hydrogen atom or a radical selected from alkyl, cycloalkyl cycloalkylalkyl, aryl and arylalkyl;
- R₂ represents:
- a radical R₂₁ which is a hydrogen atom or a radical selected from:
. an unsubstituted or substituted alkyl group,
. an unsubstituted or substituted alkenyl group,
. an unsubstituted or substituted alkynyl group, and
. an unsubstituted or substituted cycloalkyl or cycloalkylalkyl group,
- or a radical R₂₂ selected from:
. an alkylamino group in which the alkyl group may be unsubstituted or substituted, and
. a cycloalkylamino or cycloalkylalkylamino group in which the cycloalkyl or cycloalkylalkyl group may be unsubstituted or substituted; and
- Ar represents an unsubstituted or substituted group selected from:
. naphthyl,
. indolyl,
. benzofuranyl, and
. benzothiophenyl,
it being possible for Ar also to be partially or completely hydrogenated,
wherein in the description of formula (I):
- the terms "alkyl" and "alkoxy" denote linear or branched groups containing from 1 to 6 carbon atoms,
- the terms "alkenyl" and "alkynyl" denote unsaturated linear or branched groups containing from 2 to 6 carbon atoms,
- the term "cycloalkyl" denotes a group having from 3 to 8 carbon atoms,
- the term "substituted" associated with the groups alkyl, cycloalkyl, alkenyl, alkynyl and cycloalkylalkyl means that the groups may be substituted by one or more radicals selected from halogen, alkyl and alkoxy,
- the term "substituted" associated with the group Ar means that Ar is substituted by one or more radicals R, which may be the same or different, selected from halogen, hydroxy, Ra, -CH₂-Ra, -O-Ra, -CO-Ra and -O-CO-Ra (Ra being selected from alkyl, substituted alkyl, alkenyl, alkynyl, cycloalkyl, substituted cycloalkyl, cycloalkylalkyl, substituted cycloalkylalkyl, aryl, substituted aryl, arylalkyl and substituted arylalkyl), and
- the term "substituted" associated with the expressions "aryl" and "arylalkyl" means that the substitution consists of one or more radicals selected from halogen, alkyl, alkoxy, hydroxy, and alkyl substituted by one or more halogen atoms;
and their enantiomers or diastereoisomers.

2. Compounds according to claim 1 in which the group Ar is mono- or di-substituted according to one of the following formulae: 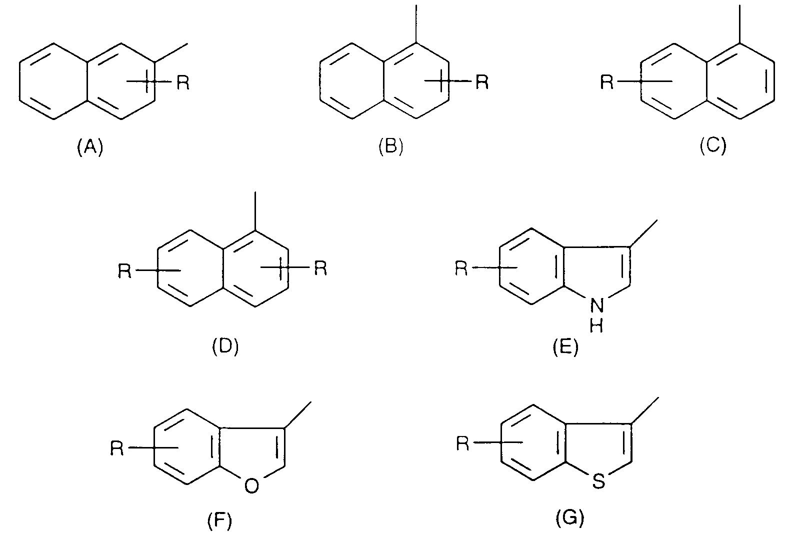 wherein R is as defined in claim 1.

3. Compounds according to claim 1 in which the group Ar is mono- or di-substituted according to one of the following formulae: 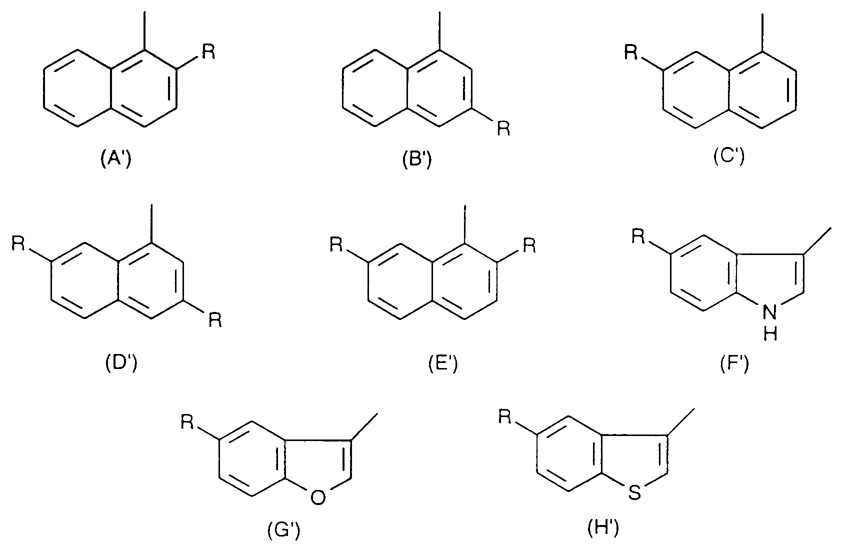 wherein R is as defined in formula (I).

4. Compound according to claims 1 to 3 which is O-[(7-methoxynaphth-1-yl)methyl]-N-acetylhydroxylamine.

5. Compound according to claims 1 to 3 which is O-[(7-methoxynaphth-1-yl)methyl]-N-butyrylhydroxylamine.

6. Compound according to claims 1 to 3 which is O-[(7-methoxynaphth-1-yl)methyl]-N-cyclopropylcarbonylhydroxylamine.

7. Compound according to claims 1 to 3 which is O-[(2-methoxynaphth-1-yl)methyl]-N-acetylhydroxylamine.

8. Process for the preparation of the compounds of formula (I), in which a compound of formula (II):
Ar-CH₂-O-NH-R₁ (II),
in which Ar and R₁ are as defined in claim 1, is reacted with a compound of formula (III): 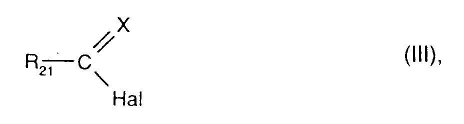 in which R₂₁ and X are as defined in claim 1 and Hal represents a halogen atom or with the corresponding acid anhydride or with formic acid, to give a compound of formula (I'): 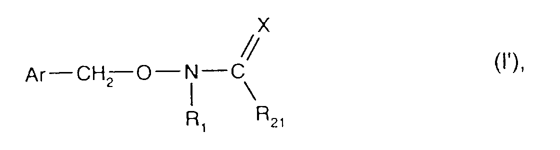 in which Ar, X, R₁ and R₂₁ are as defined above,
or with a compound of formula (III'):
X=C=N-R₂₂ (III'),
in which X and R₂₂ are as defined in claim 1, to give a compound of formula (I''): 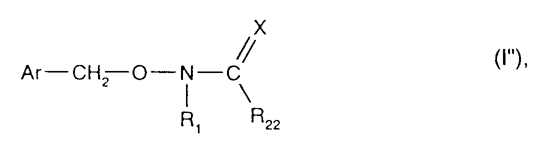 in which Ar, R₁, R₂₂ and X are as defined above,
the totality of the compounds of formulae (I') and (I'') forming the compounds of the general formula (I), which may be purified by a conventional separation method, and which are converted, if desired, into their addition salts with a pharmaceutically acceptable acid or base.

9. Pharmaceutical compositions comprising at least one compound of formula (I) according to one of claims 1 to 7 in combination with one or more pharmaceutically acceptable excipients.

10. Pharmaceutical compositions according to claim 9 for use in the prevention and treatment of disorders of the melatoninergic system.

## Patentansprüche

1. Verbindungen der Formel (I): 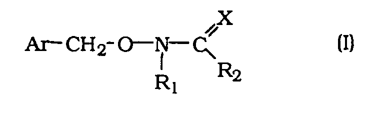 in der
- X ein Sauerstoff- oder Schwefelatom,
- R₁ ein Wasserstoffatom oder eine Gruppe ausgewählt aus Alkyl-, Cycloalkyl-. Cycloalkylalkyl-, Aryl- und Arylalkylgruppen,
- R₂:
- eine Gruppe R₂₁, welche ein Wasserstoffatom oder eine Gruppe ausgewählt aus:
• einer nichtsubstituierten oder substituierten Alkylgruppe,
• einer nichtsubstituierten oder substituierten Alkenylgruppe,
• einer nichtsubstituierten oder substituierten Alkinylgruppe und
• einer nichtsubstituierten oder substituierten Cycloalkyl- oder Cycloalkylalkylgruppe darstellt,
- oder eine Gruppe R₂₂ ausgewählt aus:
• einer Alkylaminogruppe, bei der die Alkylgruppe gegebenenfalls substituiert ist und
• einer Cycloalkylamino- oder Cycloalkylalkylaminogruppe, bei die Cycloalkyl- oder Cycloalkylalkylgruppe gegebenenfalls substituiert ist,
- Ar eine substituierte oder nichtsubstituierte Gruppe ausgewählt aus:
• Naphthyl-,
• Indolyl-,
• Benzofuranyl- und
• Benzothiophenylgruppen
worin Ar ebenfalls teilweise oder vollständig hydriert sein kann,
mit der Maßgabe bedeuten, daß bei der Definition der Formel (I):
- die Begriffe "Alkyl" und "Alkoxy" für geradkettige oder verzweigte Gruppen, die 1 bis 6 Kohlenstoffatome enthalten, stehen,
- die Begriffe "Alkenyl" und "Alkinyl" für geradkettige oder verzweigte ungesättigten Gruppen, die 2 bis 6 Kohlenstoffatome enthalten, stehen,
- der Begriff "Cycloalkyl" eine Gruppe mit 3 bis 8 Kohlenstoffatomen darstellt,
- der Begriff "substituiert", wie er für die Alkyl-, Cycloalkyl-, Alkenyl-, Alkinyl- und Cycloalkylalkylgruppen benutzt wird, bedeutet, daß die Substitution durch eine oder mehrere Gruppen ausgewählt aus Halogenatomen, Alkyl- und Alkoxygruppen erfolgen kann,
- der Begriff "substituiert", wie er für die Gruppe Ar benutzt wird, bedeutet, daß Ar durch eine oder mehrere gleichartige oder verschiedene Gruppen R substituiert ist, die ausgewählt sind aus Halogenatomen, Hydroxygruppen, Ra, -CH₂-Ra, -O-Ra, -CO-Ra und -O-CO-Ra (worin Ra aus Alkyl, substituiertem Alkyl, Alkenyl, Alkinyl, Cycloalkyl, substituiertem Cycloalkyl, Cycloalkylalkyl, substituiertem Cycloalkylalkyl, Aryl, substituiertem Aryl, Arylalkyl und substituiertem Arylalkyl ausgewählt ist), und
- der Begriff "substituiert", wie er für die Begriffe "Aryl" und "Ärylalkyl" benutzt wird, bedeutet, daß die Substitution aus einer oder mehreren Gruppen ausgewählt aus Halogenatomen, Alkylgruppen, Alkoxygruppen, Hydroxygruppen und durch ein oder mehrere Halogenatome substituierte Alkylgruppen besteht;
und deren Enantiomere oder Diastereoisomere.

2. Verbindungen nach Anspruch 1, bei denen die Gruppe Ar gemäß einer der foglenden Formeln mono- oder disubstituiert ist: 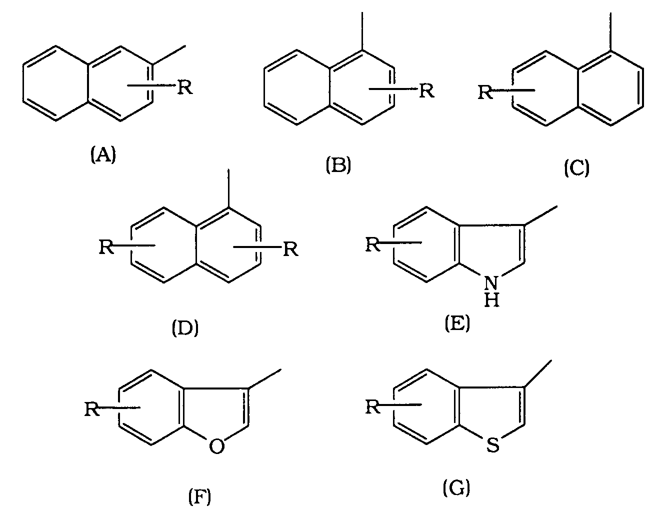 worin R die in Anspruch 1 angegebenen Bedeutungen besitzt.

3. Verbindungen nach Anspruch 1, bei denen die Gruppe Ar gemäß einer der folgenden Formeln mono- oder disubstituiert ist: 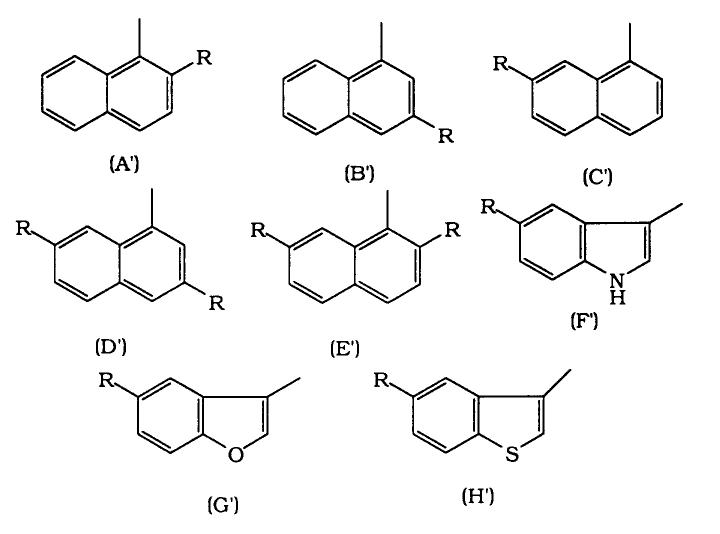 worin R die bezüglich der Formel (I) angegebenen Bedeutungen besitzt.

4. Verbindung nach den Ansprüchen 1 bis 3, nämlich O-[(7-Methoxynaphth-1-yl)-methyl]-N-acetyl-hydroxylamin.

5. Verbindung nach den Ansprüchen 1 bis 3, nämlich O-[(7-Methoxynaphth-1-yl)-methyl]-N-butyryl-hydroxylamin.

6. Verbindung nach den Ansprüchen 1 bis 3, nämlich O-[(7-Methoxynaphth-1-yl)-methyl]-N-cyclopropylcarbonyl-hydroxylamin.

7. Verbindung nach den Ansprüchen 1 bis 3, nämlich O-[(2-Methoxynaphth-1-yl)-methyl]-N-acetyl-hydroxylamin.

8. Verfahren zur Herstellung der Verbindungen der Formel (I), gemäß dem man eine Verbindung der Formel (II):
Ar ― CH₂ ― O ― NH ― R₁ (II)
in der Ar und R₁ die in Anspruch 1 angegebenen Bedeutungen besitzen, mit einer Verbindung der Formel (III): 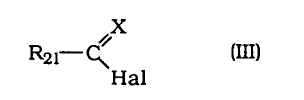 in der R₂₁ und X die in Anspruch 1 angegebenen Bedeutungen besitzen und Hal ein Halogenatom darstellt, oder einem Anhydrid der entsprechenden Säure oder mit Ameisensäure umsetzt zur Bildung einer Verbindung der Formel (I'): 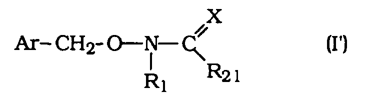 in der Ar, X, R₁ und R₂₁ die oben angegebenen Bedeutungen besitzen,
oder mit einer Verbindung der Formel (III') umsetzt:
X=C=N―R₂₂ (III)
in der X und R₂₂ die in Anspruch 1 angegebenen Bedeutungen besitzen, zur Bildung einer Verbindung der Formel (I''): 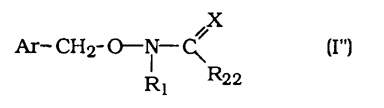 in der Ar, R₁, R₂₂ und X die oben angegebenen Bedeutungen besitzen,
wobei die Gesamtheit der Verbindungen der Formeln (I') und (II'') die Verbindungen der allgemeinen Formel (I) darstellen, welche mit Hilfe einer klassischen Trennungsmethode gereinigt werden können und welche man gewünschtenfalls mit einer pharmazeutisch annehmbaren Säure oder Base in ihre Additionssalze überführt.

9. Pharmazeutische Zubereitungen enthaltend mindestens eines Verbindung der Formel (I) nach einem der Ansprüche 1 bis 7 in Kombination mit einem oder mehreren pharmazeutisch annehmbaren Trägermaterialien.

10. Pharmazeutische Zubereitungen nach Anspruch 9 geeignet zur Vorbeugung und zur Behandlung von Störungen des melatoninergischen Systems.
